# EUROPEAN PATENT SPECIFICATION

(11) **EP 2 558 560 B1**
(45) Date of publication and mention of the grant of the patent: **14.10.2015**
(21) Application number: 11714904.7
(22) Date of filing: 06.04.2011
(51) Int. Cl.: C11D 3/386, C12N 9/54, C11D 3/37

(54) **AUTOMATIC DISHWASHING DETERGENT COMPOSITION**
SPÜLMITTELZUSAMMENSETZUNG FÜR GESCHIRRSPÜLAUTOMAT
COMPOSITION DÉTERGENTE POUR LE LAVAGE DE LA VAISSELLE EN MACHINE

(30) Priority: 20.09.2010 US 384487 P; 15.04.2010 US 324472 P
(43) Date of publication of application: 20.02.2013
(73) Proprietor: The Procter & Gamble Company, Cincinnati, OH 45202 (US)
(72) Inventor: SOUTER, Phillip, Frank, Morpeth Northumberland NE65 8UP (GB); WARD, Glenn, Steven, Newcastle upon Tyne NE3 5LL (GB); GOEDEGEBUUR, Frits, NL-3135 XV Vlaardingen (NL); POULOSE, Ayrookaran, Joseph, Belmont California 94002 (US); ESTELL, David, A., San Francisco California 94114 (US); BOTT, Richard, R., Burlingame California 94010 (US); CASCÃO-PEREIRA, Luis, Gustavo, Redwood City California 94065 (US)
(74) Representative: Yorquez Ramirez, Maria Isabel
(86) International application number: PCT/US2011/031378
(87) International publication number: WO 2011/130076

(56) References cited:
- EP-A1- 2 100 949
- WO-A1-2008/040818
- WO-A1-2010/056671
- WO-A1-2010/123754
- WO-A2-2008/153934
- WO-A2-2009/149144
- WO-A2-2010/056640
- US-B1- 6 312 936

## Description

### FIELD OF THE INVENTION

The present invention is in the field of automatic dishwashing detergent compositions, as well as methods of making and using same. In particular, it relates to an automatic dishwashing detergent composition comprising a new protease. The composition provides improved cleaning and finishing.

### BACKGROUND OF THE INVENTION

The automatic dishwashing detergent formulator is constantly looking for improved and more cost effective formulations. USPA 2009/0233831 A1 discloses an automatic dishwashing detergent composition comprising a combination of an improved protease and a low temperature amylase. Yet there is the need for better compositions.

WO2010123754 represents prior art in the sense of Article 54(3) EPC and relates to methods and compositions for the production of mature proteases in bacterial hosts. One of the proteases produced consists of SEQ ID NO:1 of the present application with the mutations N74D + S85R + G116R + S126L + P127Q + S128A + S182D + V238R.

Improved cleaning, particularly of protein-based stains such as egg stains, and shine of table ware, for example plates, cups, pots and forks, is desired. Phosphate can act as a moisture sink thereby protecting other moisture sensitive ingredients, such as enzymes, contained in the detergent and thus contribute to the aforementioned desired properties. In recent years there has been a tendency towards the elimination of phosphate from detergents. Such elimination negatively impacts the detergent's ability to clean and its shelf stability. Thus, the present challenge is to maintain/improve the detergent's stability and cleaning performance/shine-particularly when the detergent is free of phosphate.

Applicants disclose an automatic dishwashing detergent composition comprising a new protease. Such composition meets the aforementioned challenge. In one aspect, the automatic dishwashing detergent composition comprises an improved protease and an amylase and optionally a lipase. The automatic dishwashing detergent compositions of the invention prevent grit formation on washed items, which is one of the problems currently found in automatic dishwashing. The automatic dishwashing detergent composition provides excellent cleaning and finishing results even at low temperatures and it is environmentally friendly in terms of energy and raw material reduction.

### SUMMARY OF THE INVENTION

The present invention is in the field of automatic dishwashing detergent compositions, as well as methods of making and using same. In particular, it relates to an automatic dishwashing detergent composition comprising a new protease. The composition provides improved cleaning and finishing. In particular the automatic dishwashing detergent composition of the invention provides better proteinaceous removal at the same level of other proteases available in the market. This also alternatively allows for the use of a lower level of the protease of the invention and therefore a more cost effective composition.

The invention provides an automatic dishwashing detergent composition comprising a variant protease of a parent protease, said parent protease amino acid sequence being identical to the amino acid sequence of SEQ ID NO: 1, said variant protease of said parent protease mutations consisting of one of the following sets of mutations versus said parent protease:
(i) N74D + S85R + G116R + S126L + P127Q + S128A;
(ii) N74D + S85R + G116R + S126L + P127Q + S128A + S182D + V238R;
and a builder.

The invention also provides an automatic dishwashing detergent dosing element for use in an auto-dosing device, the dosing element comprising a composition according to the invention.

In a further aspect, the invention provides a method of dishwashing in an automatic dishwashing machine using an automatic dishwashing detergent composition according to the invention, comprising the step of placing the automatic dishwashing detergent composition into a product dispenser or into an auto-dosing dispensing device and releasing it during the main-wash cycle.

### BRIEF DESCRIPTION OF THE FIGURES

Figure 1 presents an alignment of the mature amino acid sequence of *B. lentus* subtilisin GG36, the mature amino acid sequence of *B. amyloliquefaciens* subtilisin BPN', and amino acid sequences of exemplary variant protease polypeptides of the invention designated as PX4, and PX5, respectively.
Figure 2 provides a plasmid map of the pHPLT-GG36 *B. subtilis* expression plasmid.

### DETAILED DESCRIPTION OF THE INVENTION

### Enzyme related terminology

### Nomenclature for amino acid modifications

In describing enzyme variants herein, the following nomenclature is used for ease of reference: Original amino acid(s):position(s):substituted amino acid(s).

According to this nomenclature, for instance the substitution of glutamic acid for glycine in position 195 is shown as G195E. A deletion of glycine in the same position is shown as G195*, and insertion of an additional amino acid residue such as lysine is shown as G195GK. Where a specific enzyme contains a "deletion" in comparison with other enzyme and an insertion is made in such a position this is indicated as *36D for insertion of an aspartic acid in position 36. Multiple mutations are separated by pluses, i.e.: S99G+V102N, representing mutations in positions 99 and 102 substituting serine and valine for glycine and asparagine, respectively. Where the amino acid in a position (*e.g.* 102) may be substituted by another amino acid selected from a group of amino acids, e.g. the group consisting of N and I, this will be indicated by V102N/I.

In all cases, the accepted IUPAC single letter or triple letter amino acid abbreviation is employed.

### Protease Amino Acid Numbering

The numbering used in this patent is the BPN' numbering system which is commonly used in the art. An alternative numbering scheme is numbering the specific amino acid sequence of the protease (GG36) listed as SEQ ID NO:1. For convenience the two different numbering schemes of two variant proteases for use in automatic dishwashing detergent compositions of the invention are compared below in Table 1:

**Table 1 - Protease Mutation numbering**

| GG36 numbering (numbering versus SEQ ID NO:1) | Equivalent BPN' numbering of this patent |
|---|---|
| N74D + S85R + G116R + S126L + P127Q + S128A | N76D + S87R + G118R + S128L + P129Q + S130A |
| N74D + S85R + G116R + S126L + P127Q + S128A + S182D + V238R | N76D + S87R + G118R + S128L + P129Q + S130A + S188D + V244R |

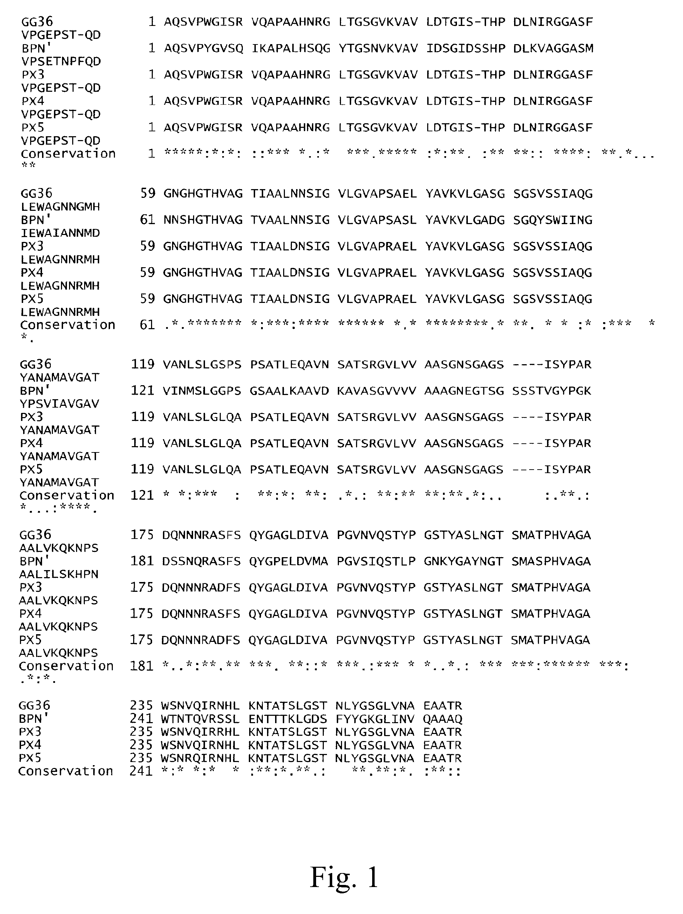

Figure 1 shows the alignment of the mature amino acid sequence of *B. lentus* subtilisin GG36, the mature amino acid sequence of *B. amyloliquefaciens* subtilisin BPN', and amino acid sequences of exemplary variant protease polypeptides of the invention designated as PX4, and PX5, respectively.

### Amino acid identity

The relatedness between two amino acid sequences is described by the parameter "identity". For purposes of the present invention, the alignment of two amino acid sequences is determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between an amino acid sequence of and enzyme used herein ("invention sequence") and a different amino acid sequence ("foreign sequence") is calculated as the number of exact matches in an alignment of the two sequences, divided by the length of the "invention sequence" or the length of the "foreign sequence", whichever is the shortest. The result is expressed in percent identity. An exact match occurs when the "invention sequence" and the "foreign sequence" have identical amino acid residues in the same positions of the overlap. The length of a sequence is the number of amino acid residues in the sequence.

The term "succinate based compound" and "succinic acid based compound" are used interchangeably herein.

As used herein, articles such as "a" and "an" when used in a claim, are understood to mean one or more of what is claimed or described.

As used herein, the terms "include", "includes" and "including" are meant to be nonlimiting.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### Automatic Dishwashing Detergent Compositions:

In one aspect, the invention provides an automatic dishwashing detergent composition comprising a variant protease of a parent protease, said parent protease amino acid sequence being identical to the amino acid sequence of SEQ ID NO:1, said variant protease of said parent protease mutations consisting of one of the following sets of mutations versus said parent protease:
(i) N74D + S85R + G116R + S126L + P127Q + S128A;
(ii) N74D + S85R + G116R + S126L + P127Q + S128A + S182D + V238R;
and a builder.

In one aspect, said automatic dishwashing detergent composition may comprise a phosphate or a non-phosphate builder and wherein the non-phosphate builder is selected from MGDA (methyl-glycine-diacetic acid); GLDA (glutamic-N,N- diacetic acid), IDS (iminodisuccinic acid), carboxy methyl inulin salts and derivatives thereof and a mixture thereof.

In one aspect, said automatic dishwashing detergent composition may comprise a sulfonated polymer.

In one aspect, said automatic dishwashing detergent composition may comprise a drying aid.

In one aspect, said automatic dishwashing detergent composition may comprise an amylase enzyme.

In one aspect, said automatic dishwashing detergent composition may comprise a cellulase enzyme.

In one aspect, said automatic dishwashing detergent composition the level of protease is from about 0.01 mg to about 5 mg, from about 0.1 mg to about 4.5 mg, or from about 0.5 mg to about 4 mg of active protease per gram of composition.

### Detailed Description of Additional Materials For Use In Automatic Dishwashing Detergent Composition

### Additional proteases

In the automatic dishwashing detergent composition of the invention a mixture of two or more proteases may be used. A mixture of proteases can contribute to an enhanced cleaning across a broader temperature and/or substrate range and provide superior shine benefits, especially when used in conjunction with a sulfonated polymer.

Suitable proteases for use in combination with the variant protease used in the invention include metalloproteases and serine proteases, including neutral or alkaline microbial serine proteases, such as subtilisins (EC 3.4.21.62). Suitable proteases include those of animal, vegetable or microbial origin. Microbial origin is preferred. Chemically or genetically modified mutants are included. The protease may be a serine protease, in one aspect, an alkaline microbial protease or a chymotrypsin or trypsin-like protease. Examples of neutral or alkaline proteases include:
(a) subtilisins (EC 3.4.21.62), especially those derived from Bacillus, such as Bacillus lentus, B. alkalophilus, B. subtilis, B. amyloliquefaciens, Bacillus pumilus and Bacillus gibsonii described in US 6,312,936 B1, US 5,679,630, US 4,760,025, and USPA 2009/0170745A1.
(b) trypsin-like or chymotrypsin-like proteases, such as trypsin (*e.g.,* of porcine or bovine origin), the Fusarium protease described in USP 5,288,627 and the chymotrypsin proteases derived from Cellumonas described in USPA 2008/0063774A1.
(c) metalloproteases, especially those derived from Bacillus amyloliquefaciens described in USPA 2009/0263882A1 and USPA 2008/0293610A1.

Suitable commercially available protease enzymes include those sold under the trade names Alcalase®, Savinase®, Primase®, Durazym®, Polarzyme®, Kannase®, Liquanase®, Ovozyme®, Neutrase®, Everlase® and Esperase® by Novozymes A/S (Denmark), those sold under the tradename Maxatase®, Maxacal®, Maxapem®, Properase®, Purafect®, Purafect Prime®, Purafect Ox®, FN3®, FN4®, Excellase® and Purafect OXP® by Genencor International (now Danisco US Inc.), and those sold under the tradename Opticlean® and Optimase® by Solvay Enzymes, those available from Henkel/ Kemira, namely BLAP (sequence shown in Figure 29 of US 5,352,604 with the following mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D) - all from Henkel/Kemira; and KAP (Bacillus alkalophilus subtilisin with mutations A230V + S256G + S259N) from Kao.

In one aspect, commercial proteases selected from the group consisting of Properase®, Purafect®, Ovozyme®, Everlase®, Savinase®, Excellase® and FN3® are employed.

### Amylases

Amylase enzymes are additional enzymes that are useful in Applicants' automatic dish washing detergent composition. Suitable amylases include those described in USPA 2009/0233831 A1 and USPA 2009/0314286A1. Suitable commercially available amylases for use herein include STAINZYME®, STAINZYME PLUS®, STAINZYME ULTRA® and NATALASE® (Novozymes A/S) and Spezyme Xtra™ and Powerase™. STAINZYME PLUS® and Powerase™ may be particularly useful.

### Cellulases

In one aspect, the automatic dishwashing detergent composition of the invention comprises a cellulase enzyme. This composition provides excellent results in terms of not only cleaning of the dishware/tableware but also in terms of cleaning of the dishwasher. Cellulase enzymes include microbial-derived endoglucanases exhibiting endo-beta-1,4-glucanase activity (E.C. 3.2.1.4), including a bacterial polypeptide endogenous to a member of the genus Bacillus which has a sequence of at least 90%, 94%, 97% and even 99% identity to the amino acid sequence SEQ ID NO:2 in US 7,141,403B2) and mixtures thereof. Suitable commercially available cellulases for use herein include Celluzyme®, Celluclean®, Whitezyme® (Novozymes A/S) and Puradax HA® (Genencor International - now Danisco US Inc.).

### Other Additional enzymes

Other additional enzymes suitable for use in the automatic dishwashing detergent composition of the invention can comprise one or more enzymes selected from the group comprising hemicellulases, cellobiose dehydrogenases, peroxidases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, ß-glucanases, arabinosidases, hyaluronidase, chondroitinase, laccase, and mixtures thereof.

In one aspect,, such additional enzyme may be selected from the group consisting of lipases, including "first cycle lipases" comprising a substitution of an electrically neutral or negatively charged amino acid with R or K at any of positions 3, 224, 229, 231 and 233 on the wild-type of Humicola Lanuginosa, whose sequence is shown as SEQ ID No 1 in pages 5 and 6 of U.S. Patent 6,939,702 B1, in one aspect, a variant comprising T231R and N233R mutations. One such variant is sold under the tradename Lipex® (Novozymes A/S, Bagsvaerd, Denmark).

Enzyme stabilizer components - Suitable enzyme stabilizers include oligosaccharides, polysaccharides and inorganic divalent metal salts, such as alkaline earth metal salts, especially calcium salts. Chlorides and sulphates are may be particularly suitable with calcium chloride, in one aspect, being an especially suitable calcium salt. Examples of suitable oligosaccharides and polysaccharides, such as dextrins, can be found in USPA 2008/0004201 A1. In case of aqueous compositions comprising protease, a reversible protease inhibitor, such as a boron compound, inckuding borate and 4-formyl phenyl boronic acid or a tripeptide aldehyde, can be added to further improve stability.

### Cleaning actives

Any cleaning ingredient in addition to builders can be used as part of the automatic dishwashing detergent product of the invention. The levels given are weight per cent and refer to the total composition (excluding the enveloping water-soluble material, in the case of unit dose forms having a wrapper or enveloping material). The automatic dishwashing detergent composition can contain a phosphate builder or be free of phosphate builder and comprise one or more detergent active components which may be selected from bleach, bleach activator, bleach catalyst, surfactants, alkalinity sources, sulfonated polymer, dying aids, anti-corrosion agents (e.g. sodium silicate) and care agents. Particularly suitable cleaning components for use herein include a builder compound, a bleach, an alkalinity source, a surfactant, an anti-scaling polymer for example, a sulfonated polymer, an enzyme and an additional bleaching agent.

### Surfactant

Surfactants suitable for use herein include non-ionic surfactants. Traditionally, non-ionic surfactants have been used in automatic dishwashing detergent compositions for surface modification purposes in particular for sheeting to avoid filming and spotting and to improve shine. It has been found that non-ionic surfactants can also contribute to prevent redeposition of soils.

In one aspect, the automatic dishwashing detergent product of the invention comprises is a non-ionic surfactant or a non-ionic surfactant system, in one aspect, the non-ionic surfactant or a non-ionic surfactant system has a phase inversion temperature, as measured at a concentration of 1% in distilled water, between 40 °C and 70°C, preferably between 45 °C and 65°C. A "non-ionic surfactant system" means a mixture of two or more non-ionic surfactants. Non-ionic surfactant systems are typically especially useful as they seem to have improved cleaning and finishing properties and better stability in product than single non-ionic surfactants.

Phase inversion temperature is the temperature below which a surfactant, or a mixture thereof, partitions preferentially into the water phase as oil-swollen micelles and above which it partitions preferentially into the oil phase as water swollen inverted micelles. Phase inversion temperature can be determined visually by identifying at which temperature cloudiness occurs.

The phase inversion temperature of a non-ionic surfactant or system can be determined as follows: a solution containing 1% of the corresponding surfactant or mixture by weight of the solution in distilled water is prepared. The solution is stirred gently before phase inversion temperature analysis to ensure that the process occurs in chemical equilibrium. The phase inversion temperature is taken in a thermostable bath by immersing the solutions in 75 mm sealed glass test tube. To ensure the absence of leakage, the test tube is weighed before and after phase inversion temperature measurement. The temperature is gradually increased at a rate of less than 1°C per minute, until the temperature reaches a few degrees below the pre-estimated phase inversion temperature. Phase inversion temperature is determined visually at the first sign of turbidity.

Suitable nonionic surfactants include: i) ethoxylated non-ionic surfactants prepared by the reaction of a monohydroxy alkanol or alkyphenol with 6 to 20 carbon atoms typically with at least 12 moles, at least 16 moles, or even at least 20 moles of ethylene oxide per mole of alcohol or alkylphenol; ii) alcohol alkoxylated surfactants having a from 6 to 20 carbon atoms and at least one ethoxy and propoxy group. In one aspect, mixtures of surfactants i) and ii) are particularly useful.

Another class of suitable non-ionic surfactants are epoxy-capped poly(oxyalkylated) alcohols represented by the formula:

R¹O[CH₂CH(CH₃)O]ₓ[CH₂CH₂O]_{y}[CH₂CH(OH)R²] (I)

wherein R¹ is a linear or branched, aliphatic hydrocarbon radical having from 4 to 18 carbon atoms; R² is a linear or branched aliphatic hydrocarbon radical having from 2 to 26 carbon atoms; x is an integer having an average value of from 0.5 to 1.5, or about 1; and y is an integer having a value of at least 15, or at least 20.

In one aspect, the surfactant of formula I, at least about 10 carbon atoms in the terminal epoxide unit [CH₂CH(OH)R²]. Suitable surfactants of formula I, according to the present invention, include Olin Corporation's POLY-TERGENT® SLF-18B nonionic surfactants, as described, for example, in USP 5,766,371 and USP 5,576,281.

Suitable non-ionic surfactants and/or system to use as anti-redeposition agents herein may have a Draves wetting time of less than 360 seconds, less than 200 seconds, less than 100 seconds or less than 60 seconds as measured by the Draves wetting method (standard method ISO 8022 using the following conditions; 3-g hook, 5-g cotton skein, 0.1% by weight aqueous solution at a temperature of 25°C).

Amine oxides surfactants are also useful in the present invention as anti-redeposition surfactants and include linear and branched compounds having the formula: 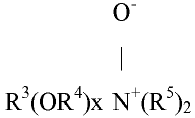 wherein R³ is selected from an alkyl, hydroxyalkyl, acylamidopropoyl and alkyl phenyl group, or mixtures thereof, containing from 8 to 26 carbon atoms, or 8 to 18 carbon atoms; R⁴ is an alkylene or hydroxyalkylene group containing from 2 to 3 carbon atoms, or 2 carbon atoms, or mixtures thereof; x is from 0 to 5, or from 0 to 3; and each R⁵ is an alkyl or hydroxyalkyl group containing from 1 to 3, or from 1 to 2 carbon atoms, or a polyethylene oxide group containing from 1 to 3, or even 1, ethylene oxide group. The R⁵ groups can be attached to each other, e.g., through an oxygen or nitrogen atom, to form a ring structure.

These amine oxide surfactants in particular include C₁₀-C₁₈ alkyl dimethyl amine oxides and C₈-C₁₈ alkoxy ethyl dihydroxyethyl amine oxides. Examples of such materials include dimethyloctylamine oxide, diethyldecylamine oxide, bis-(2-hydroxyethyl)dodecylamine oxide, dimethyldodecylamine oxide, dipropyltetradecylamine oxide, methylethylhexadecylamine oxide, dodecylamidopropyl dimethylamine oxide, cetyl dimethylamine oxide, stearyl dimethylamine oxide, tallow dimethylamine oxide and dimethyl-2-hydroxyoctadecylamine oxide. In one aspect, C₁₀-C₁₈ alkyl dimethylamine oxide, and C₁₀-C₁₈ acylamido alkyl dimethylamine oxide are employed.

Surfactants may be present in amounts from 0 to 10% by weight, from 0.1 % to 10%, and or even from 0.25% to 6% by weight of the total composition.

### Builder

Builders for use herein include phosphate builders and phosphate free builders. If present, builders are used in a level of from 5% to 60%, from 10% to 50%, or even from 10% to 50% by weight of the automatic dishwashing detergent composition. In some embodiments the automatic dishwashing detergent product comprises a mixture of phosphate and non-phosphate builders.

### Phosphate builders

Preferred phosphate builders include mono-phosphates, di-phosphates, tripolyphosphates or oligomeric-poylphosphates are used. The alkali metal salts of these compounds are preferred, in particular the sodium salts. An especially preferred builder is sodium tripolyphosphate (STPP).

### Non-phosphate builders

Useful non-phosphate builders include amino acid based compounds, in particular MGDA (methyl-glycine-diacetic acid), and salts and derivatives thereof, GLDA (glutamic-N,N-diacetic acid) and salts and derivatives thereof, IDS (iminodisuccinic acid) and salts and derivatives thereof, carboxy methyl inulin and salts and derivatives thereof and mixtures thereof. In one aspect, GLDA (salts and derivatives thereof) is especially useful, with the tetrasodium salt thereof being especially useful. In one aspect, MGDA or GLDA are present in the automatic dishwashing detergent composition of the invention in a level of from 0.5% to 20%, from about 1% to about 10% or from about 2 to about 7% by weight of the composition.

Suitable builders for use herein, in addition or instead of MGDA and/or GLDA, include builders which form water-soluble hardness ion complexes (sequestering builder) such as citrates and builders which form hardness precipitates (precipitating builder) such as carbonates e.g. sodium carbonate.

Other suitable non-phosphate builders include amino acid based compound or a succinate based compound. Other suitable builders are described in USP 6,426,229. In one aspect, suitable builders include; for example, aspartic acid-N-monoacetic acid (ASMA), aspartic acid-N,N-diacetic acid (ASDA), aspartic acid-N- monopropionic acid (ASMP), iminodisuccinic acid (IDA), N- (2-sulfomethyl) aspartic acid (SMAS), N- (2-sulfoethyl) aspartic acid (SEAS), N- (2-sulfomethyl) glutamic acid (SMGL), N- (2- sulfoethyl) glutamic acid (SEGL), N-methyliminodiacetic acid (MIDA), alpha- alanine-N,N-diacetic acid (alpha -ALDA), serine-N,N-diacetic acid (SEDA), isoserine-N,N-diacetic acid (ISDA), phenylalanine-N,N-diacetic acid (PHDA), anthranilic acid- N ,N - diacetic acid (ANDA), sulfanilic acid-N, N-diacetic acid (SLDA), taurine-N, N-diacetic acid (TUDA) and sulfomethyl-N,N-diacetic acid (SMDA) and alkali metal salts or ammonium salts thereof.

In one aspect, the non-phosphate builder may be present in the automatic dishwashing detergent composition in an amount of at least 1% , at least 5%, at least 10%, or at least 20% by weight of the total composition. In one aspect, these builders are present in an amount of up to 50%, up to 45%, up to 40%, or up to 35% by weight of the total composition. In certain aspects the composition contains 20% by weight of the total composition or less of phosphate builders, 10% by weight of the total composition or less, or the composition is substantially free of phosphate builders.

Other non-phosphate builders include homopolymers and copolymers of polycarboxylic acids and their partially or completely neutralized salts, monomeric polycarboxylic acids and hydroxycarboxylic acids and their salts. In one aspect, salts of the abovementioned compounds include the ammonium and/or alkali metal salts, i.e. the lithium, sodium, and potassium salts, and sodium salts may be particularly useful.

Suitable polycarboxylic acids include acyclic, alicyclic, heterocyclic and aromatic carboxylic acids, in which case they contain at least two carboxyl groups which are in each case separated from one another, in one aspect by no more than two carbon atoms. Polycarboxylates which comprise two carboxyl groups include, for example, water-soluble salts of, malonic acid, (ethyl enedioxy) diacetic acid, maleic acid, diglycolic acid, tartaric acid, tartronic acid and fumaric acid. Polycarboxylates which contain three carboxyl groups include, for example, water-soluble citrate. Correspondingly, a suitable hydroxycarboxylic acid is, for example, citric acid. Another suitable polycarboxylic acid is the homopolymer of acrylic acid. Other suitable builders are disclosed in USP 5,698,504.

### Sulfonated polymer

The polymer, if present, is used in any suitable amount from about 0.1% to about 50%, from 0.5% to about 20%, or from 1% to 10% by weight of the automatic dishwashing detergent composition. Sulfonated/carboxylated polymers are particularly suitable for the automatic dishwashing detergent composition of the invention.

Suitable sulfonated/carboxylated polymers described herein may have a weight average molecular weight of less than or equal to about 100,000 Da, less than or equal to about 75,000 Da, less than or equal to about 50,000 Da, from about 3,000 Da to about 50,000 Da, or from about 5,000 Da to about 45,000 Da.

As noted herein, the sulfonated/carboxylated polymers may comprise (a) at least one structural unit derived from at least one carboxylic acid monomer having the general formula (I): 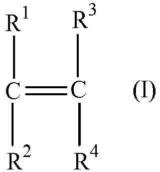 wherein R¹ to R⁴ are independently hydrogen, methyl, carboxylic acid group or CH₂COOH and wherein the carboxylic acid groups can be neutralized; (b) optionally, one or more structural units derived from at least one nonionic monomer having the general formula (II): 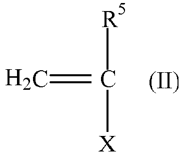 wherein R⁵ is hydrogen, C₁ to C₆ alkyl, or C₁ to C₆ hydroxyalkyl, and X is either aromatic (with R⁵ being hydrogen or methyl when X is aromatic) or X is of the general formula (III): 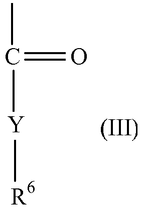 wherein R⁶ is (independently of R⁵) hydrogen, C₁ to C₆ alkyl, or C₁ to C₆ hydroxyalkyl, and Y is O or N; and at least one structural unit derived from at least one sulfonic acid monomer having the general formula (IV): 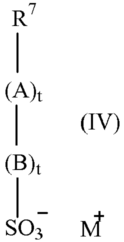 wherein R⁷ is a group comprising at least one sp2 bond, A is O, N, P, S or an amido or ester linkage, B is a mono- or polycyclic aromatic group or an aliphatic group, each t is independently 0 or 1, and M+ is a cation. In one aspect, R₇ is a C₂ to C₆ alkene. In another aspect, R⁷ is ethene, butene or propene.

Suitable carboxylic acid monomers include one or more of the following: acrylic acid, maleic acid, itaconic acid, methacrylic acid, or ethoxylate esters of acrylic acids, acrylic and methacrylic acids being more preferred. In one aspect, sulfonated monomers include one or more of the following: sodium (meth) allyl sulfonate, vinyl sulfonate, sodium phenyl (meth) allyl ether sulfonate, or 2-acrylamido-methyl propane sulfonic acid. In one aspect, non-ionic monomers include one or more of the following: methyl (meth) acrylate, ethyl (meth) acrylate, t-butyl (meth) acrylate, methyl (meth) acrylamide, ethyl (meth) acrylamide, t-butyl (meth) acrylamide, styrene, or α-methyl styrene.

In one aspect, the polymer comprises the following levels of monomers: from about 40% to about 90%, from about 60% to about 90% by weight of the polymer of one or more carboxylic acid monomer; from about 5% to about 50%, from about 10% to about 40% by weight of the polymer of one or more sulfonic acid monomer; and optionally from about 1% to about 30%, from about %2 to about 20% by weight of the polymer of one or more non-ionic monomer. An especially suitable polymer comprises about 70% to about 80% by weight of the polymer of at least one carboxylic acid monomer and from about 20% to about 30% by weight of the polymer of at least one sulfonic acid monomer.

The carboxylic acid is may be (meth)acrylic acid. The sulfonic acid monomer is typically one of the following: 2-acrylamido methyl-1-propanesulfonic acid, 2-methacrylamido-2-methyl-1-propanesulfonic acid, 3-methacrylamido-2-hydroxypropanesulfonic acid, allysulfonic acid, methallysulfonic acid, allyloxybenzenesulfonic acid, methallyloxybenzensulfonic acid, 2-hydroxy-3-(2-propenyloxy)propanesulfonic acid, 2-methyl-2-propene-1-sulfonic acid, styrene sulfonic acid, vinylsulfonic acid, 3-sulfopropyl acrylate, 3-sulfopropyl methacrylate, sulfomethylacrylamid, sulfomethylmethacrylamide, and water soluble salts thereof. The unsaturated sulfonic acid monomer is , in one aspect, 2-acrylamido-2-propanesulfonic acid (AMPS).

Commercial available polymers include: Alcosperse 240, Aquatreat AR 540 and Aquatreat MPS supplied by Alco Chemical; Acumer 3100, Acumer 2000, Acusol 587G and Acusol 588G supplied by Rohm & Haas; Goodrich K-798, K-775 and K-797 supplied by BF Goodrich; and ACP 1042 supplied by ISP technologies Inc. Particularly suitable polymers are Acusol 587G and Acusol 588G supplied by Rohm & Haas.

In the polymers, all or some of the carboxylic or sulfonic acid groups can be present in neutralized form, i.e. the acidic hydrogen atom of the carboxylic and/or sulfonic acid group in some or all acid groups can be replaced with metal ions, for example alkali metal ions and in particular sodium ions.

### Drying aids

In another embodiment, the automatic dishwashing detergent composition of the invention comprises a drying aid. By "drying aid" herein is meant an agent capable of decreasing the amount of water left on washed items, in particular in plastic items that are more prone to be wet after the washing process due to their hydrophobic nature.

Suitable drying aids include polyesters, especially anionic polyesters derived from terephthalic acid, 5-sulphoisophthalic acid or a salt of 5-sulphoisophthalic, ethyleneglycol or polyethyleneglycol, propyleneglycol or polypropyleneglycol, and, polyalkyleneglycol monoalkylethers, optionally together with further monomers with 3 to 6 functionalities which are conducive to polycondensation, specifically acid, alcohol or ester functionalities. Suitable polyesters to use as drying aids are disclosed in WO 2008/110816 and preferably have one or more of the following properties:
(a) a number average molecular weight of from about 800 Da to about 25,000 Da, or from about 1,200 Da to about 12,000 Da.
(b) a softening point greater than about 40°C from about 41°C to about 200°C, or even 80°C to about 150°C;
(c) a solubility greater than about 6% by weight in water of 3 ° German hardness at 200°C. At 30°C the solubility will typically be greater than about 8% by weight, at 40°C and 50°C , the solubility will typically be greater than about 40% by as measured in water of 3 ° German hardness.

Other suitable drying aids include specific polycarbonate-, polyurethane- and/or polyurea-polyorganosiloxane compounds or precursor compounds thereof of the reactive cyclic carbonate and urea type, as described in USPA 2010/0041574 A1 and USPA 2010/0022427 A1.

Improved drying can also be achieved by use of non-ionic surfactants, such as:
(a) R¹O[CH₂CH(CH₃)O]ₓ[CH₂CH₂O]_{y}[CH₂CH(CH₃)O]_{z}CH₂CH(OH)R², in which R¹ represents a linear or branched aliphatic hydrocarbon radical having 4 to 22 carbon atoms or mixtures thereof and R² represents a linear or branched hydrocarbon radical having 2 to 26 carbon atoms or mixtures thereof, x and z represent integers from 0 to 40, and y represents a integer of at least 15, or from 15 to 50. See for example as in WO 2009/033972; or
(b) RO[CHCH(R^{a})O]ᵢ[CH₂CH_{2O}]ₘ[CH₂CH(R¹)O]ₙC(O)R² where R is a branched or unbranched alkyl radical having 8 to 16 carbon atoms, R^{a} and R¹ independently of one another, are hydrogen or a branched or unbranched alkyl radical having 1 to 5 carbon atoms, R² is an unbranched alkyl radical having 5 to 17 carbon atoms; 1 and n are independently of one another, an integer from 1 to 5 and m is an integer from 13 to 35, as described in USPA 2008/016721.

Examples of suitable materials include Plurafac LF731 or Plurafac LF-7319 (BASF) and the Dehyquart® CSP and Polyquart^{®} range (Cognis).

In one aspect, these non-ionic surfactants are used in combination with one or more of:
(a) a sulphonated polymer; or
(b) alkoxylated alcohols, particularly alkyl ethoxylates wherein the alkyl chain has from 8 to 14 carbon atoms, with an average of from 4 to 10, or from 6 to 8 ethoxylates, such as Lutensol TO7 supplied by BASF.

In one aspect, the automatic dishwashing detergent composition of the invention comprises from about 0.1% to about 10%, from about 0.5% to about 5% and especially from about 1% to about 4% by weight of the composition of a drying aid.

### Silicates

Suitable silicates are sodium silicates such as sodium disilicate, sodium metasilicate and crystalline phyllosilicates. Silicates if present are at a level of from about 1% to about 20%, or from about 5% to about 15% by weight of the automatic dishwashing detergent composition.

### Bleach

Inorganic and organic bleaches are suitable cleaning actives for use herein. Inorganic bleaches include perhydrate salts such as perborate, percarbonate, perphosphate, persulfate and persilicate salts. The inorganic perhydrate salts are normally the alkali metal salts. The inorganic perhydrate salt may be included as the crystalline solid without additional protection. Alternatively, the salt can be coated.

Alkali metal percarbonates, particularly sodium percarbonate are preferred perhydrates for use herein. The percarbonate is most preferably incorporated into the products in a coated form which provides in-product stability. A suitable coating material providing in product stability comprises mixed salt of a water-soluble alkali metal sulphate and carbonate. Such coatings together with coating processes have previously been described in USP 4,105,827. The weight ratio of the mixed salt coating material to percarbonate lies in the range from 1: 200 to 1: 4, from 1: 99 to 1 9, or from 1: 49 to 1: 19. In one aspect, the mixed salt is of sodium sulphate and sodium carbonate which has the general formula Na₂SO₄.n.Na₂CO₃ wherein n is from 0.1 to 3, from 0.2 to 1.0 or from 0.2 to 0.5.

Another suitable coating material providing in product stability, comprises sodium silicate of SiO₂: Na₂O ratio from 1.8: 1 to 3.0: 1, or L8:1 to 2.4:1, and/or sodium metasilicate, in one aspect, applied at a level of from 2% to 10%, (normally from 3% to 5%) of SiO₂ by weight of the inorganic perhydrate salt. Magnesium silicate can also be included in the coating. Coatings that contain silicate and borate salts or boric acids or other inorganics are also suitable.

Other coatings which contain waxes, oils, fatty soaps can also be used advantageously within the present invention.

Potassium peroxymonopersulfate is another inorganic perhydrate salt of utility herein.

Typical organic bleaches are organic peroxyacids including diacyl and tetraacylperoxides, especially diperoxydodecanedioc acid, diperoxytetradecanedioc acid, and diperoxyhexadecanedioc acid. Dibenzoyl peroxide is a preferred organic peroxyacid herein. Mono- and diperazelaic acid, mono- and diperbrassylic acid, and Nphthaloylaminoperoxicaproic acid are also suitable herein.

The diacyl peroxide, especially dibenzoyl peroxide, should typically be present in the form of particles having a weight average diameter of from about 0.1 to about 100 microns, from about 0.5 to about 30 microns, or from about 1 to about 10 microns.In one aspect,, at least about 25%, at least about 50%, at least about 75%, or at least about 90%, of the particles are smaller than 10 microns, or smaller than 6 microns. Diacyl peroxides within the above particle size range have also been found to provide better stain removal especially from plastic dishware, while minimizing undesirable deposition and filming during use in automatic dishwashing machines, than larger diacyl peroxide particles. The optimum diacyl peroxide particle size thus allows the formulator to obtain good stain removal with a low level of diacyl peroxide, which reduces deposition and filming. Conversely, as diacyl peroxide particle size increases, more diacyl peroxide is needed for good stain removal, which increases deposition on surfaces encountered during the dishwashing process.

Further typical organic bleaches include the peroxy acids, particular examples being the alkylperoxy acids and the arylperoxy acids. Preferred representatives are (a) peroxybenzoic acid and its ring-substituted derivatives, such as alkylperoxybenzoic acids, but also peroxy-α-naphthoic acid and magnesium monoperphthalate, (b) the aliphatic or substituted aliphatic peroxy acids, such as peroxylauric acid, peroxystearic acid, ε-phthalimidoperoxycaproic acid[phthaloiminoperoxyhexanoic acid (PAP)], o-carboxybenzamidoperoxycaproic acid, N-nonenylamidoperadipic acid and N-nonenylamidopersuccinates, and (c) aliphatic and araliphatic peroxydicarboxylic acids, such as 1,12-diperoxycarboxylic acid, 1,9-diperoxyazelaic acid, diperoxysebacic acid, diperoxybrassylic acid, the diperoxyphthalic acids, 2-decyldiperoxybutane-1,4-dioic acid, N,N-terephthaloyldi(6-aminopercaproic acid).

### Bleach activators

Bleach activators are typically organic peracid precursors that enhance the bleaching action in the course of cleaning at temperatures of 60° C and below. Bleach activators suitable for use herein include compounds which, under perhydrolysis conditions, give aliphatic peroxoycarboxylic acids having from 1 to 10 carbon atoms, in particular from 2 to 4 carbon atoms, and/or optionally substituted perbenzoic acid. Suitable substances bear O-acyl and/or N-acyl groups of the number of carbon atoms specified and/or optionally substituted benzoyl groups. Preference is given to polyacylated alkylenediamines, in particular tetraacetylethylenediamine (TAED), acylated triazine derivatives, in particular 1,5-diacetyl-2,4-dioxohexahydro-1,3,5-triazine (DADHT), acylated glycolurils, in particular tetraacetylglycoluril (TAGU), N-acylimides, in particular N-nonanoylsuccinimide (NOSI), acylated phenolsulfonates, in particular n-nonanoyl- or isononanoyloxybenzenesulfonate (n- or iso-NOBS), carboxylic anhydrides, in particular phthalic anhydride, acylated polyhydric alcohols, in particular triacetin, ethylene glycol diacetate and 2,5-diacetoxy-2,5-dihydrofuran and also triethylacetyl citrate (TEAC). Bleach activators if included in the automatic dishwashing detergent compositions of the invention are in a level of from about 0.1% to about 10%, or from about 0.5% to about 2% by weight of the total composition.

### Bleach catalyst

Bleach catalysts preferred for use herein include the manganese triazacyclononane and related complexes (US-A-4246612, US-A-5227084); Co, Cu, Mn and Fe bispyridylamine and related complexes (US-A-5114611); and pentamine acetate cobalt(III) and related complexes(US-A-4810410). A complete description of bleach catalysts suitable for use herein can be found in USP 6,599,871, pages 34, line 26 to page 40, line 16. Bleach catalyst if included in the automatic dishwashing detergent compositions of the invention are in a level of from about 0.1% to about 10%, or from about 0.5% to about 2% by weight of the total composition.

### Metal care agents

Metal care agents may prevent or reduce the tarnishing, corrosion or oxidation of metals, including aluminium, stainless steel and non-ferrous metals, such as silver and copper. Suitable examples include one or more of the following:
(a) benzatriazoles, including benzotriazole or bis-benzotriazole and substituted derivatives thereof. Benzotriazole derivatives are those compounds in which the available substitution sites on the aromatic ring are partially or completely substituted. Suitable substituents include linear or branch-chain C₁-C₂₀- alkyl groups and hydroxyl, thio, phenyl or halogen such as fluorine, chlorine, bromine and iodine.
(b) metal salts and complexes chosen from the group consisting of zinc, manganese, titanium, zirconium, hafnium, vanadium, cobalt, gallium and cerium salts and/or complexes, the metals being in one of the oxidation states II, III, IV, V or VI. In one aspect, suitable metal salts and/or metal complexes may be chosen from the group consisting of Mn(II) sulphate, Mn(II) citrate, Mn(II) stearate, Mn(II) acetylacetonate, K₂TiF₆, K₂ZrF₆, CoSO₄, Co(NO₃)₂ and Ce(NO₃)₃, zinc salts, for example zinc sulphate, hydrozincite or zinc acetate.;
(c) silicates, including sodium or potassium silicate, sodium disilicate, sodium metasilicate, crystalline phyllosilicate and mixtures thereof.

Further suitable organic and inorganic redox-active substances that act as silver/copper corrosion inhibitors are disclosed in USP 5,888,954.

In one aspect, the automatic dishwashing detergent composition of the invention comprises from 0.1% to 5%, from 0.2% to 4% or from 0.3% to 3% by weight of the total composition of a metal care agent. In one aspect, the metal care agent comprises a zinc salt.

### Method of Use

A method of dishwashing in an automatic dishwashing machine using any aspect of Applicants' automatic dishwashing detergent composition disclosed in the present specification is disclosed, said method comprising the step of placing said automatic dishwashing detergent composition into a product dispenser or into an auto-dosing dispensing device and releasing it during the main-wash cycle.

### Unit dose form

In one aspect, a unit dose form comprising, from about 10 grams to about 25 grams or from about 12 grams to about 24 grams of any aspect of Applicants' automatic dishwashing detergent composition disclosed in the present specification is disclosed.

In one aspect, an automatic dishwashing detergent dosing element for use in an auto-dosing device the dosing element comprising any aspect of Applicants' automatic dishwashing detergent composition disclosed in the present specification is disclosed.

### Additional Detailed Unit Dose Disclosure

In one aspect, the automatic dishwashing detergent composition of the invention is in unit dose form. Automatic dishwashing detergent products in unit dose form include tablets, capsules, sachets, pouches, etc. In one aspect, for use herein are tablets wrapped with a water-soluble film and water-soluble pouches. The weight of the composition of the invention is from about 10 to about 25 grams, from about 12 to about 24 grams or even from 14 to 22 grams. These weights are extremely convenient for automatic dishwashing detergent product dispenser fit. In the cases of unit dose products having a water-soluble material enveloping the automatic dishwashing detergent composition, the water-soluble material is not considered as part of the composition.

In one aspect, the unit dose form is a water-soluble pouch (i.e., water-soluble film enveloping an automatic dishwashing detergent composition), in one aspect, a multi-compartment pouch having a plurality of films forming a plurality of compartments. This configuration contributes to the flexibility and optimization of the composition. It allows for the separation and controlled release of different ingredients. In one aspect, one compartment contains an automatic dishwashing detergent composition in solid form and another compartment contains an automatic dishwashing detergent composition in liquid form.

In one aspect, multi-compartment pouch embodiments two different compartments could contain two different cleaning agents. In one aspect, the films of these two compartments have different dissolution profiles, allowing the release of the same or different agents at different times. For example, the agent from one compartment (first compartment) can be delivered early in the washing process to help with soil removal and a second agent from another compartment (second compartment) can be delivered at least two minutes, or even at least five minutes later than the agent from the first compartment.

In one aspect, a multi-compartment pouch comprising two side-by-side compartments superposed onto another compartment wherein at least two different compartments contain two different automatic dishwashing detergent compositions is disclosed.

According to another aspect of the invention, there is provided an automatic dishwashing detergent dosing element for use in an auto-dosing device the dosing element comprising an automatic dishwashing detergent composition according to any of the preceding claims. By "auto-dosing device" herein is meant a device that is placed into the dishwasher holding a plurality of doses to be delivered in different washes. The user does not need to charge the detergent for each wash, the auto-dosing device delivers them automatically. Each wash can use a single or more doses.

A multi-compartments pack is formed by a plurality of water-soluble enveloping materials which form a plurality of compartments, one of the compartments would contain the automatic dishwashing detergent composition of the invention, another compartment can contain a liquid composition, the liquid composition can be aqueous (i.e. comprises more than 10% of water by weight of the liquid composition) and the compartment can be made of warm water soluble material. In some embodiments the compartment comprising the automatic dishwashing detergent composition of the invention is made of cold water soluble material. It allows for the separation and controlled release of different ingredients. In other embodiments all the compartments are made of warm water soluble material.

Suitable packs comprise at least two side-by-side compartments superposed (i.e. placed above) onto another compartment, especially suitable are pouches. This disposition contributes to the compactness, robustness and strength of the pack, additionally, it minimises the amount of water-soluble material required. It only requires three pieces of material to form three compartments. The robustness of the pack allows also for the use of very thin films without compromising the physical integrity of the pack. The pack is also very easy to use because the compartments do not need to be folded to be used in machine dispensers of fix geometry. At least two of the compartments of the pack contain two different automatic dishwashing detergent compositions. By "different compositions" herein is meant automatic dishwashing detergent compositions that differ in at least one ingredient.

In one aspect, at least one of the compartments contains a solid automatic dishwashing detergent composition and another compartment an aqueous liquid automatic dishwashing detergent composition, the compositions are typically in a solid to liquid weight ratio of from about 20:1 to about 1:20, from about 18:1 to about 2:1 or from about 15:1 to about 5:1. This kind of pack is very versatile because it can accommodate compositions having a broad spectrum of values of solid:liquid ratio. Pouches having a high solid:liquid ratio because many of the detergent ingredients are particularly suitable for use in solid form, in one aspect in powder form. The ratio solid:liquid defined herein refers to the relationship between the weight of all the solid compositions and the weight of all the liquid compositions in the pack.

Suitable solid:liquid weight ratios are from about 2:1 to about 18:1, or from about 5:1 to about 15:1. These weight ratios are suitable in cases in which most of the ingredients of the detergent are in liquid form.

In one aspect, the two side-by-side compartments contain liquid automatic dishwashing detergent compositions, which can be the same or different and another compartment contains a solid automatic dishwashing detergent composition, for example in powder form, in one aspect, a densified powder. The solid composition contributes to the strength and robustness of the pack.

For dispenser fit reasons, especially in an automatic dishwasher, the unit dose form products herein have a square or rectangular base and a height of from about 1 to about 5 cm, or from about 1 to about 4 cm. In one aspect, the weight of the solid composition is from about 5 to about 20 grams, or from about 10 to about 15 grams and the weight of the liquid compositions is from about 0.5 to about 4 grams, or from about 0.8 to about 3 grams.

In one aspect, at least two of the films which form different compartments have different solubilities, under the same conditions. This enables the release of the compositions which they partially or totally envelope at different times.

Controlled release of the ingredients of a multi-compartment pouch can be achieved by modifying the thickness of the film and/or the solubility of the film material. The solubility of the film material can be delayed by for example cross-linking the film as described in USPA 2002/0198125A1. Other water-soluble films designed for rinse release are described in US 4,765,916 and US 4,972,017. Waxy coating (see USP 5,453,216) of films can help with rinse release. pH controlled release means are described in USP 5,453,216, in particular amino-acetylated polysaccharide having selective degree of acetylation.

Other means of obtaining delayed release by multi-compartment pouches with different compartments, where the compartments are made of films having different solubility are taught in USP 6 727,215.

### Auto-dosing delivery device

The dosing elements of the present invention can be placed into a delivery cartridge. The dosing elements can have an elongated shape and set into an array forming a delivery cartridge which is the refill for an auto-dosing dispensing device. The delivery cartridge is to be placed in an auto-dosing delivery device. Suitable disclosure of auto-dosing can be found in USPA 2009/0170743 A1, USPA 2008/0293604 A1, USPA 2009/0308414 A1 and USPA 2010/0065084 A1.

### EXPERIMENTAL

The following examples are provided in order to demonstrate and further illustrate certain embodiments and aspects of the present invention and are not to be construed as limiting the scope thereof.

In the experimental disclosure which follows and elsewhere herein, the following abbreviations apply: PI (proteinase inhibitor), ppm (parts per million); M (molar); mM (millimolar); µM (micromolar); nM (nanomolar); mol (mole); mmol (millimole); µmol (micromole); nmol (nanomole); gm (gram); mg (milligram); µg (microgram); pg (picogram); L or 1 (liter); ml and mL (milliliters); µl or µL (microliter); cm (centimeter); mm (millimeter); µm (micrometer); nm (nanometer); U (units); V (volt); MW (molecular weight); sec (second); min(s) (minute/minutes); h(s) or hr(s) (hour/hours); °C (degrees Centigrade); ND (not determined); rpm (revolutions per minute); GH (degrees German hardness); H₂O (water); dH₂O (deionized water); HCl (hydrochloric acid); aa (amino acid); bp (base pair); kb (kilobase pair); kD (kilodaltons); cDNA (copy or complementary DNA); DNA (deoxyribonucleic acid); ssDNA (single stranded DNA); dsDNA (double stranded DNA); RNA (ribonucleic acid); MgCl₂ (magnesium chloride); NaCl (sodium chloride); BPN' (*Bacillus amyloliquefaciens* subtilisin); PB92 (*Bacillus clausii* subtilisin); w/v (weight to volume); v/v (volume to volume); w/w (weight to weight); g (gravity); OD (optical density); ppm (parts per million); OD₂₈₀ (optical density at 280 nm); OD₆₀₀ (optical density at 600 nm); A₄₀₅ (absorbance at 405 nm); PAGE (polyacrylamide gel electrophoresis); PBS (phosphate buffered saline [150 mM NaCl, 10 mM sodium phosphate buffer, pH 7.2]); PEG (polyethylene glycol); PCR (polymerase chain reaction); SDS (sodium dodecyl sulfate); TRIS or Tris (tris(hydroxymethyl)aminomethane); HEPES (N-[2-Hydroxyethyl]piperazine-N-[2-ethanesulfonic acid]); HBS (HEPES buffered saline); Tris-HCl (tris[Hydroxymethyl]aminomethane-hydrochloride); DMSO (dimethyl sulfoxide); SA (sinapinic acid (s,5-dimethoxy-4-hydroxy cinnamic acid); TCA (trichloroacetic acid); HPLC (high pressure liquid chromatography); *Taq* (*Thermus aquaticus* DNA polymerase); Klenow (DNA polymerase I large (Klenow) fragment); EDTA (ethylenediaminetetracetic acid); bla (β-lactamase or ampicillin-resistance gene); HDL (high density liquid); HDD (heavy duty powder detergent); HSG (high suds granular detergent); CEE (Central and Eastern Europe); WE (Western Europe); NA, when used in reference to detergents (North America); Japan and JPN, when used in reference to detergents (Japan); CFT (Center for Test Materials, Vlaardingen, the Netherlands); P&G and Procter & Gamble (Procter & Gamble, Inc., Cincinnati, OH); DNA2.0 (DNA2.0, Menlo Park, CA); Corning (Corning Life Sciences, Corning, NY); ATCC (American Type Culture Collection, Rockville, MD); Sigma (Sigma Chemical Co., St. Louis, MO); NCBI (National Center for Biotechnology Information); Operon Technologies (Operon Technologies, Inc., Alameda, CA); Invitrogen (Invitrogen Corp., San Diego, CA); Qiagen (Qiagen, Inc., Valencia, CA); Molecular Devices (Molecular Devices Corp., Sunnyvale, CA); Siegfried Handel (Siegfried Handel AG, Zofingen, Switzerland); Stratagene (Stratagene Cloning Systems, La Jolla, CA); Monsanto (Monsanto Co., St. Louis, MO); Wintershall (Wintershall AG, Kassel, Germany); BASF (BASF Co., Florham Park, NJ); Huntsman (Huntsman Petrochemical Corp., Salt Lake City, UT); Enichem (Enichem Iberica, Barcelona, Spain); Fluka Chemie AG (Fluka Chemie AG, Buchs, Switzerland); Gist-Brocades (Gist-Brocades, NV, Delft, the Netherlands); Dow Corning (Dow Corning Corp., Midland, MI); RB (Reckitt-Benckiser, Slough, UK).

### EXAMPLE 1

### Construction of Variant Proteases

Variant proteases PX4 and PX5 can be created by using one or more of a variety of standard methods well known to those of ordinary skill in the art. For example, a nucleic acid encoding the PX4 or PX5 variant protease can be constructed by performing standard site-directed mutagenesis of a plasmid DNA encoding a *B. lentus* GG36 protease-encoding nucleotide sequence. A GG36 protease-encoding nucleotide sequence is as follows:

This DNA sequence comprises a nucleotide sequence encoding a signal peptide (shown above in non-underlined, lowercase letters), a nucleotide sequence encoding a propeptide (shown above in underlined, lower-case letters), and a nucleotide sequence encoding mature GG36 polypeptide (shown above in uppercase letters).

The amino acid sequence of the mature variant protease referred to herein as PX4 with amino acid substitutions N76D/S87R/G118R/S128L/P129Q/S130A relative to SEQ ID NO:1 (using BPN' numbering determined by alignment of the PX4 polypeptide sequence with the BPN' polypeptide sequence shown in SEQ ID NO:2) is:

The amino acid sequence of the mature variant protease referred to herein as PX5 with amino acid substitutions N76D/S87R/G118R/S128L/P129Q/S130A/S188D/V244R relative to SEQ ID NO:1 (using BPN' numbering determined by alignment of the PX5 polypeptide sequence with the BPN' polypeptide sequence shown in SEQ ID NO:2) is:

Exemplary site-directed mutagenesis procedures well known in the art include, but are not limited to, e.g., the QuikChange® Multi Site-Directed Mutagenesis method embodied in the QuikChange® Multi Site-Directed Mutagenesis Kit (QCMS; Agilent Technologies - Stratagene, La Jolla, CA), which allows for site-directed mutagenesis of plasmid DNA at up to five different sites simultaneously. Nucleic acids encoding the PX4 and PX5 variant proteases can also be readily made from, e.g., the GG36 protease-encoding nucleotide sequence by one skilled in the art using well-known gene synthesis methods and/or fusion PCR methods (see, e.g., U.S.P.A. 2006/0252155).

Nucleic acids encoding the PX4 and PX5 variant proteases can also be made by chemical synthesis using, e.g., the classical phosphoramidite method (see, e.g., Beaucage et al., Tetrahedron Letters 22:1859-69 (1981)) or the method described by Matthes et al., EMBO J. 3:801-05 (1984), e.g., as is typically practiced in automated synthesis methods. Alternatively, nucleic acids encoding the PX4 and PX5 variant proteases can be ordered from a variety of commercial sources, such as from The Midland Certified Reagent Company (Midland, Texas) (worldwide website address at oligos.com), the Great American Gene Company (worldwide website address genco.com), Operon Technologies, Inc. (Alameda, Calif.) (now Qiagen, see worldwide website at qiagen.com), or DNA2.0 (Menlo Park, CA). Other techniques for synthesizing nucleic acids and related principles are described in, e.g., Itakura et al., Annu. Rev. Biochem. 53:323 (1984) and Itakura et al., Science 198:1056 (1984).

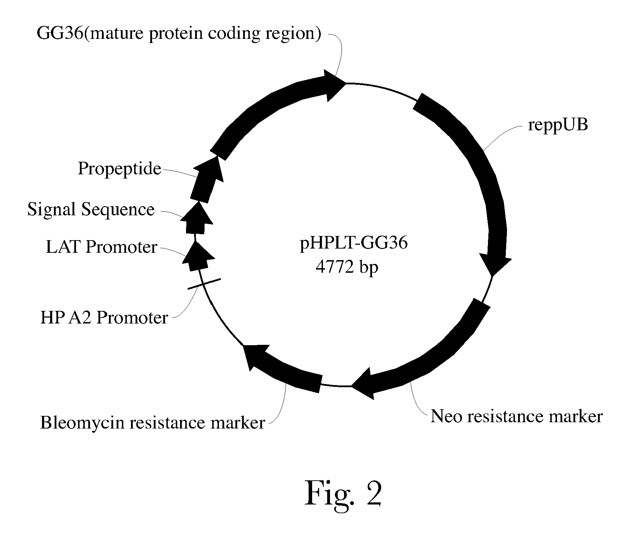

In one aspect, for example, if gene synthesis is used to create the PX4- or PX5-encoding nucleic acid, such nucleic acid can be designed with flanking restriction sites such as, e.g., BglII, which can be used to clone the PX4- or PX5-encoding nucleic acid into an expression plasmid (e.g., *B. subtilis* expression plasmid) also digested with BglII, such as pHPLT-GG36 *B. subtilis* expression plasmid described herein. This exemplary pHPLT *B. subtilis* expression vector contains the *B. licheniformis* LAT promoter (Plat), HPA2 promoter, and additional elements from pUB110 (see, e.g., McKenzie et al., Plasmid, 15:93-103 (1986)), including a replicase gene (reppUB), a neomycin resistance gene (neo), and a bleomycin resistance marker (bleo) (see also Figure 4 of U.S. Patent No. 6,566,112). The pHPLT-GG36 plasmid map is provided at Figure 2, and the GG36 expression cassette sequence is provided below. For the QuikChange^{®} Multi Site-Directed Mutagenesis Kit (QCMS Kit) or fusion PCR methods described herein, the pHPLT-GG36 plasmid comprising the *B. lentus* GG36-encoding nucleic acid can be used as the DNA template for making the PX4 and PX5 variant proteases used in the invention. In an exemplary format, nucleotide primers containing the desired mutations of PX4 or PX5 are annealed to the GG36-encoding nucleic acid in the pHPLT-GG36 plasmid and extended with a DNA polymerase as described in the Stratagene QCMS product manual and in U.S.P.A. 2006/0252155 for fusion PCR. Table 1-1 provides exemplary nucleotide sequences of the primers that can be used for site-directed mutagenesis.

| **Table 1-1. Exemplary Primers Used for QuikChange® Multi Site-Directed Mutagenesis Method** | |
|---|---|
| **Primer Sequence** | **Primer Name** |
| CGGGACGATTGCTGCTTTA**GAC**AATTCGATTGGCGTTC (SEQ ID NO:12) | N76D |
| GGCGTTCTTGGCGTAGCGCCG**AAC**GCGGAACTATACG (SEQ ID NO:13) | S87N |
| CCAAGGATTGGAATGGGCAGGGAACAAT**CGT**ATGCACGTTG (SEQ ID NO:14) | G118R |
| TAATTTGAGTTTAGGA**CTGCAGGCA**CCAAGTGCCACACTTGAGC (SEQ ID NO:15) | S128L,P129Q,S130A |
| CCAAAACAACAACCGCGCC**GAT**TTTTCACAGTATGGCGC (SEQ ID NO:16) | S188D |
| ATCTTGGTCCAAT**CGT**CAAATCCGCAATCATCTAAAGAATACGGC (SEQ ID NO:17) | V244R |

The incorporation of mutations in each PX4 and PX5 variant protease can be carried out in multiple rounds till the final variant protease is obtained. Rolling circle amplification (GE Healthcare, Piscataway, NJ) can be used as described by the manufacturer to amplify the mutant plasmids contained in the QCMS or fusion PCR ligation reactions before transformation in *B. subtilis* (GE Healthcare, Piscataway, NJ) cells.

Competent *B. subtilis* cells (phenotype: Δ*aprE,* Δ*nprE, oppA, ΔspoIIE, degUHy32, ΔamyE*::(*xylR,pxylA-comK*)) can be transformed with the variant plasmids or 1 µL of the rolling circle amplification reaction to obtain protease positive transformants using procedures known in the art (see, e.g., WO 02/14490). The bacteria can be made competent by the induction of the *comK* gene under control of a xylose inducible promoter (see, e.g., Hahn et al., Mol. Microbiol. 21:763-775 (1996)). Variant protease positive clones can be selected on skim milk/agar plates, isolated, sequenced and variant protease protein produced in shaker flask cultures to generate significant quantities of enzyme samples for characterization.

### EXAMPLE 2

### Production of Variant Proteases in Bacillus subtilis

The variant proteases were produced by growing the *B. subtilis* transformants overnight at 37°C in 10ml TSB (tryptone and soy based broth) medium. A 250µl aliquot of the overnight culture was transferred into 25ml of a MOPS based defined medium in a 100ml shake flask and grown at 37°C for 68 hours. The defined medium was made essentially as known in the art (*See,* Neidhardt et al., J Bacteriol, 119: 736-747, 1974), except that NH₄Cl, FeSO₄, and CaCl₂ were left out of the base medium, 3 mM K₂HPO₄ was used, and the base medium was supplemented with 60 mM urea, 75 g/L glucose, and 1% soytone. Also the micronutrients were made up as a 100X stock containing in one liter, 400 mg FeSO₄.7H₂O, 100 mg MnSO₄.H₂O, 100 mg ZnSO₄.7H₂O, 50 mg CuCl₂.2H₂O, 100 mg CoCl₂.6H₂O, 100 mg NaMoO₄.2H₂O, 100 mg Na₂B₄O₇.10H₂O, 10 ml of 1M CaCl₂, and 10 ml of 0.5 M sodium citrate. The proteases of interest were isolated from the culture medium.

### Abbreviations used in Example 3

In the example, the abbreviated component identifications have the following meanings:

| | |
|---|---|
| Carbonate | : Anhydrous sodium carbonate |
| STPP | : Sodium tripolyphosphate anhydrous |
| Silicate | : Amorphous Sodium Silicate (SiO₂:Na₂O = from 2:1 to 4:1) |
| Alcosperse 240-D | : Sulfonated polymer available from Alco Chemical 95% solids |
| Percarbonate | : Sodium percarbonate of the nominal formula 2Na₂CO₃.3H₂O₂ |
| TAED | : Tetraacetylethylenediamine |
| Detergency enzyme | available from Novozymes A/S |
| SLF18 | : Non-ionic surfactant available from BASF |
| Lutensol TO7 | Non-ionic surfactant available from BASF |
| LF224 | Non-ionic surfactant available from BASF |
| Neodol 1-9 | : Non-ionic surfactant available from Shell |
| DPG | : dipropylene glycol |

In the following example all levels are quoted in per cent by weight of the composition (either solid or liquid composition).

### Example 3

The automatic dishwashing detergent compositions tabulated below are introduced into a multi-compartment pouch having a first compartment comprising the solid automatic dishwashing detergent composition (in powder form) and a liquid compartment superposed onto the powder compartment comprising the liquid automatic dishwashing detergent compositions. The film used is Monosol M8630 film as supplied by Monosol. The weight of the solid composition is 17 grams and the weight of liquid compositions is 2.6 gram.

The pouch comprises 0.5 - 2 mg of active protease per gram of automatic dishwashing detergent composition. Said protease being a variant protease of a parent protease, said parent protease's sequence being at least 97%, at least 99% or 100% identical to the amino acid sequence of SEQ ID NO:1, said variant protease of said parent protease comprising one of the following sets of mutations versus said parent protease:
(i) N76D + S87R + G118R + S128L + P129Q + S130A, with the proviso that said variant protease does not comprise the set of mutations S188D + N248R;
(ii) N76D + S87R + G118R + S128L + P129Q + S130A + S188D + V244R.

| Formulation | 1 | 2 | 3 | 4 |
|---|---|---|---|---|
| Ingredient | Level (%wt) | Level (%wt) | Level (%wt) | Level (%wt) |
| Solid automatic dishwashing detergent composition | | | | |
| STPP | 35 | 0 | 0 | 56 |
| Carbonate | 24 | 45 | 40 | 18.5 |
| Methylglycine diacetic acid (83% active) | 0 | 15 | 20 | 0 |
| Silicate | 7 | 7 | 7 | 1.5 |
| TAED | 0.5 | 0.5 | 0.5 | 3.8 |
| Zinc carbonate | 0.5 | 0.5 | 0.5 | 0 |
| SLF18 | 1.5 | 1.5 | 1.5 | 0 |
| Plurafac LF224 | | | | 0.6 |
| Penta Amine Acetato-cobalt(III) nitrate (1% active) | 0.5 | 0.5 | 0.5 | 0.6 |
| Percarbonate | 15 | 15 | 15 | 11 |
| Sulphonated polymer² | 10 | 4 | 3 | 5.1 |
| Amylase (14.4mg/g active)¹ | 1.3 | 1.8 | 1.5 | 0.7 |
| Processing aids, perfume and sodium sulphate | To balance | To balance | To balance | To balance |
| Liquid automatic dishwashing detergent composition | | | | |
| DPG | 45 | 45 | 45 | 25 |
| SLF18 | 45 | 45 | 45 | 0 |
| Neodol 1-9 | 3 | 3 | 3 | 2.6 |
| Lutensol TO7 | | | | 30 |
| Plurafac LF224 | | | | 32.4 |
| Amine Oxide | | | | 3.6 |
| Glycerine | 2 | 2 | 2 | 4 |
| Processing aids and Dyes | To balance | To balance | To balance | To balance |
| Second Liquid automatic dishwashing detergent composition* | | | | |
| Lutensol TO7 | | | | 65 |
| LF224 | | | | 32 |
| Processing aids, Dyes & Glycerine | To balance | To balance | To balance | To balance |

| | | | | |
|---|---|---|---|---|
| * Where a second liquid automatic dishwashing detergent composition is present this is as part of a 3-compartment unit dose (one powder and two liquids) ¹ Suitable amylases can be purchased from Novozymes, e.g. amylase sold under tradename Stainzyme Plus® or from Genencor, sold under tradename Powerase®. ² Suitable sulphonated polymers can be purchased from Akzo Nobel, e.g. Acusol 240-D or Acusol 588G. | | | | |

The exemplified pouch is used to wash a soiled load as described herein below in an automatic dishwasher under the conditions described herein below. The washing items present excellent shine.

### Substrates/Soils

- Corning ware round casserole dish with egg.
   ∘ 1 part of butter with 50cc of egg in microwave 4 ½ minutes.
   ∘ 2 casserole dishes per run
- Stainless steel pot
   o Painted with 10 grams of cooked and blended Kraft Macaroni and cheese
   o Baked in over for seven minutes
   ∘ 2 stainless steel pots per run
- China Vertex plate
   o Painted with five grams of cooked and blended Minute Rice
   o Dry overnight
   ∘ 2 plates per run
- Black Ceramic Plates
   o Painted with 5 grams of a composite soil (TMD) comprising eggs, vegetables, meat, and cereals.
   ∘ Allowed to dry over night
   ∘ 4 plates per run
      ▪ TMD soil is made by J&R.
- Stainless Steel Spatulas
   o Painted with five grams of TMD soil
   o Allowed to dry overnight
   ∘ 4 spatulas per run

### Test Conditions:

- Bank of four machines GE2600
- City Water (8gpg)
- Four products
- 120°F Inlet Water temperature
- Normal cycle/heated dry
- Substrates listed above are placed in the dishwasher
- 50 grams of the TMD soil is added when the main wash cup opens

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An automatic dishwashing detergent composition comprising a variant protease of a parent protease, said parent protease amino acid sequence being identical to the amino acid sequence of SEQ ID NO:1, said variant protease of said parent protease mutations consisting of one of the following sets of mutations versus said parent protease:
(i) N74D + S85R + G116R + S126L + P127Q + S128A;
(ii) N74D + S85R + G116R + S126L + P127Q + S128A + S182D + V238R;
and a builder

2. The automatic dishwashing detergent composition according to claim 1 wherein the builder comprises a phosphate or a non-phosphate builder and wherein the non-phosphate builder is selected from MGDA (methyl-glycine-diacetic acid); GLDA (glutamic-N,N-diacetic acid), IDS (iminodisuccinic acid), carboxy methyl inulin salts and derivatives thereof and a mixture thereof.

3. The automatic dishwashing detergent composition according to any of claims 1 or 2 further comprising a sulfonated polymer.

4. The automatic dishwashing detergent composition according to any of the preceding claims further comprising a drying aid.

5. The automatic dishwashing detergent composition according to any of the preceding claims further comprising an amylase enzyme.

6. The automatic dishwashing detergent composition according to any of the preceding claims further comprising a cellulase enzyme.

7. The automatic dishwashing detergent composition according to any of the preceding claims wherein the level of protease is from about 0.01 mg to about 5 mg of active protease per gram of composition.

8. The automatic dishwashing detergent composition according to any of the preceding claims wherein the composition is in unit dose form and wherein the weight of the composition is from about 10 grams to about 25 grams.

9. An automatic dishwashing detergent dosing element for use in an auto-dosing device the dosing element comprising a composition according to any of the preceding claims.

10. A method of dishwashing in an automatic dishwashing machine using an automatic dishwashing detergent composition according to any of the preceding claims comprising the step of placing the automatic dishwashing detergent composition into a product dispenser or into an auto-dosing dispensing device and releasing it during the main-wash cycle.

## Patentansprüche

1. Reinigungszusammensetzung für Geschirrspülautomaten, umfassend eine Variantenprotease einer Stammprotease, wobei die Aminosäuresequenz der Stammprotease mit der Aminosäuresequenz von SEQ ID NO: 1 identisch ist, wobei die Variantenprotease der Stammproteasemutationen aus einem der folgenden Sets von Mutationen gegenüber der Stammprotease besteht:
(i) N74D + S85R + G116R + S126L + P127Q + S128A;
(ii) N74D + S85R + G116R + S126L + P127Q + S128A + S182D + V238R;
und einen Builder (Gerüststoff).

2. Reinigungszusammensetzung für Geschirrspülautomaten nach Anspruch 1, wobei der Builder einen Phosphat- oder einen Nichtphosphatbuilder umfasst und wobei der Nichtphosphatbuilder ausgewählt ist aus MGDA (Methylglycindiessigsäure); GLDA (Glutamin-N,N-diessigsäure), IDS (Iminodibernsteinsäure), Carboxymethylinulinsalzen und Derivaten davon und einer Mischung davon.

3. Reinigungszusammensetzung für Geschirrspülautomaten nach Anspruch 1 oder 2, ferner umfassend ein sulfoniertes Polymer.

4. Reinigungszusammensetzung für Geschirrspülautomaten nach einem der vorstehenden Ansprüche, ferner umfassend ein Trocknungshilfsmittel.

5. Reinigungszusammensetzung für Geschirrspülautomaten nach einem der vorstehenden Ansprüche, ferner umfassend ein Amylaseenzym.

6. Reinigungszusammensetzung für Geschirrspülautomaten nach einem der vorstehenden Ansprüche, ferner umfassend ein Cellulaseenzym.

7. Reinigungszusammensetzung für Geschirrspülautomaten nach einem der vorstehenden Ansprüche, wobei der Proteasegehalt etwa 0,01 mg bis etwa 5 mg aktive Protease pro Gramm der Zusammensetzung beträgt.

8. Reinigungszusammensetzung für Geschirrspülautomaten nach einem der vorstehenden Ansprüche, wobei die Zusammensetzung in Einheitsdosisform vorliegt und das Gewicht der Zusammensetzung etwa 10 Gramm bis etwa 25 Gramm beträgt.

9. Reinigungsmitteldosierelement für Geschirrspülautomaten zur Verwendung in einer automatischen Dosiervorrichtung, wobei das Dosierelement eine Zusammensetzung nach einem der vorstehenden Ansprüche umfasst.

10. Verfahren zum Reinigen von Geschirr in einer automatischen Geschirrspülmaschine unter Verwendung einer Reinigungszusammensetzung für Geschirrspülautomaten nach einem der vorstehenden Ansprüche, umfassend den Schritt des Platzierens der Reinigungszusammensetzung für Geschirrspülautomaten in einem Produktspender oder einer automatischen Dosierspendevorrichtung und des Freisetzens derselben während des Hauptreinigungsgangs.

## Revendications

1. Composition détergente pour lave-vaisselle automatique comprenant une protéase variante d'une protéase mère, la séquence d'acides aminés de ladite protéase mère étant identique à la séquence d'acides aminés SEQ ID NO: 1, ladite protéase variante des mutations de ladite protéase mère consistant en l'un des ensembles suivants de mutations par rapport à ladite protéase mère :
(i) N74D + S85R + G116R + S126L + P127Q + S128A ;
(ii) N74D + S85R + G116R + S126L + P127Q + S128A + S182D + V238R ;
et un adjuvant.

2. Composition détergente pour lave-vaisselle automatique selon la revendication 1, dans laquelle l'adjuvant comprend un phosphate ou un adjuvant non phosphate, et dans laquelle l'adjuvant non phosphate est choisi parmi le MGDA (acide méthylglycine-diacétique), le GLDA (acide glutamique-N,N-diacétique), l'IDS (acide iminodisuccinique), les sels d'inuline carboxyméthylique et leurs dérivés et leurs mélanges.

3. Composition détergente pour lave-vaisselle automatique selon l'une quelconque des revendications 1 ou 2, comprenant en outre un polymère sulfoné.

4. Composition détergente pour lave-vaisselle automatique selon l'une quelconque des revendications précédentes, comprenant en outre un adjuvant de séchage.

5. Composition détergente pour lave-vaisselle automatique selon l'une quelconque des revendications précédentes, comprenant en outre une enzyme amylase.

6. Composition détergente pour lave-vaisselle automatique selon l'une quelconque des revendications précédentes, comprenant en outre une enzyme cellulase.

7. Composition détergente pour lave-vaisselle automatique selon l'une quelconque des revendications précédentes, dans lequel le niveau de protéase est d'environ 0,01 mg à environ 5 mg de protéase active par gramme de composition.

8. Composition détergente pour lave-vaisselle automatique selon l'une quelconque des revendications précédentes, dans laquelle la composition est sous forme de dose unitaire et dans laquelle le poids de la composition est d'environ 10 grammes à environ 25 grammes.

9. Élément de dosage de détergent pour lave-vaisselle automatique pour une utilisation dans un dispositif d'autodosage de l'élément de dosage comprenant une composition selon l'une quelconque des revendications précédentes.

10. Procédé de lavage de la vaisselle dans un lave-vaisselle automatique en utilisant une composition détergente pour lave-vaisselle automatique selon l'une quelconque des revendications précédentes, comprenant les étapes consistant à placer la composition détergente pour lave-vaisselle automatique dans un distributeur de produit ou dans un dispositif de distribution à autodosage et à la libérer pendant le cycle de lavage principal.


### SEQUENCE LISTING

<110> Procter & Gamble
<120> AUTOMATIC DISHWASHING DETERGENT COMPOSITION
<130> 11688L
<160> 2
<170> PatentIn version 3.5
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 2
   <211> 275
   <212> PRT
   <213> Bacillus amyloliquefaciens
<400> 2